# EUROPÄISCHE PATENTSCHRIFT

(11) **EP 2 691 157 B1**
(45) Veröffentlichungstag und Bekanntmachung des Hinweises auf die Patenterteilung: **07.06.2023**
(21) Anmeldenummer: 12710517.9
(22) Anmeldetag: 27.03.2012
(51) Int. Cl.: A61Q 19/00, A61K 8/37

(54) **ISOPENTYLESTER ZUR VERWENDUNG IN KOSMETISCHEN, DERMATOLOGISCHEN ODER PHARMAZEUTISCHEN KOMPOSITIONEN**
ISOPENTYL ESTERS FOR THE USE IN COSMETIC, DERMATOLOGICAL, OR PHARMACEUTICAL COMPOSITIONS
ESTERS D'ISOPENTYLE À UTILISER DANS DES COMPOSITIONS COSMÉTIQUES, DERMATOLOGIQUES OU PHARMACEUTIQUES

(30) Priorität: 29.03.2011 DE 102011006362
(43) Veröffentlichungstag der Anmeldung: 05.02.2014
(73) Patentinhaber: Evonik Operations GmbH, 45128 Essen (DE)
(72) Erfinder: THUM, Oliver, 40880 Ratingen (DE); ECKSTEIN, Marrit, Friederike, 45289 Essen (DE); SPRINGER, Oliver, 46485 Wesel (DE); WIECHERS, Susann, 45239 Essen (DE); MEYER, Juergen, 45134 Essen (DE)
(74) Vertreter: Evonik Patent Association
(86) Internationale Anmeldenummer: PCT/EP2012/055362
(87) Internationale Veröffentlichungsnummer: WO 2012/130820

(56) Entgegenhaltungen:
- EP-A1- 2 243 517
- EP-A2- 2 327 756
- US-A- 4 732 810
- US-A1- 2004 187 379

## Beschreibung

### Gebiet der Erfindung

Die Erfindung betrifft die Ester einer Mischung aus 2-Methyl-1-butanol und 3-Methyl-1-butanol und deren Verwendung zur Herstellung von Formulierungen.

### Stand der Technik

Kosmetische, dermatologische oder pharmazeutische Formulierungen werden in großen Mengen hergestellt und von Verbrauchern weltweit konsumiert. In letzter Zeit werden dabei verstärkt Anforderungen an die Nachhaltigkeit der Formulierungen und ihrer Inhaltsstoffe gestellt. Insbesondere besteht der Bedarf, möglichst viele der eingesetzten Komponenten auf Basis nachwachsender Rohstoffe herzustellen.

Gleichzeitig steigen aber auch die Ansprüche der Konsumenten an die sensorischen Eigenschaften der zu verwendenden Formulierungen, insbesondere die haptischen (z. B. Öligkeit, Wachsigkeit) und olfaktorischen Eigenschaften. Insbesondere gilt dies für Formulierungen, die einen hohen Anteil an Wirkstoffen dispergiert (beispielsweise anorganische UV-Filter wie TiO₂ oder ZnO) oder gelöst aufweisen (z. B. organische UV-Filter oder Harnstoff).

EP2243517A1 beschreibt die Verwendung von Estern des Isoamylalkohols (3-Methyl-1-butanol) insbesondere von Laurinsäureestern des 3-Methyl-1-butanols, in ausgewählten Sonnenschutzformulierungen, d. h. in kosmetischen Formulierungen, anorganische UV-Filter und/oder organische UV-Filter aus der Gruppe der sogenannten "Triazine", enthaltend. Dabei wird beschrieben, dass solche Sonnenschutzformulierungen ein verbessertes Hautgefühl im Vergleich zu Formulierungen auf Basis etablierter Komponenten, z. B. auf Basis von Butylene Glycol Estern oder C12-C15 Alkylbenzoat, aufweisen.

EP2243517A1 beschreibt allerdings nicht beispielhaft, welche konkreten sensorischen Eigenschaften durch die Verwendung der erfindungsgemäßen Ester verbessert werden. Weiterhin werden ausdrücklich nur Sonnenschutzformulierungen erwähnt.

"Emollients in Personal Care for Solubilizing Organic UV Absorbers" (IP.COM Journal, IP.COM Inc., West Henrietta, NY, US, 22. Februar 2006, XP013112931, ISSN 1533-0001) offenbart ebenfalls einige Komponenten die zur Herstellung von Sonnenschutzformulierungen mit guter Verteilbarkeit auf und gutem Einziehvermögen in Haut geeignet sind, beispielsweise Isoamyllaurat (vgl. S. 8). Auch hier werden ausdrücklich nur Sonnenschutzformulierungen erwähnt.

Es besteht weiterhin Bedarf an Komponenten für kosmetische, dermatologische oder pharmazeutische Formulierungen, die verbesserte sensorische Eigenschaften eben dieser Formulierungen bewirken.

Eine Aufgabe der vorliegenden Erfindung bestand daher darin, neuartige kosmetische Inhaltsstoffe zu entwickeln, welche Herstellung von kosmetischen, dermatologischen oder pharmazeutischen Formulierungen mit verbesserten sensorische Eigenschaften erlauben.

### Beschreibung der Erfindung

Überraschenderweise wurde gefunden, dass Ester auf Basis eines Gemisches von 3-Methyl-1-butanol und 2-Methyl-1-butanol diese Anforderungen erfüllen. Solch ein Isomerengemisch des Isopentylalkohols kann aus den Rückständen der alkoholischen Gärung gewonnen werden und stellt daher einen natürlichen Rohstoff dar.

Im Vergleich zu Formulierungen auf Basis bekannter Inhaltsstoffe, z. B. Ester des reinen 3-Methyl-1-butanols, zeigen die erfindungsgemäßen Formulierungen eine geringeres öliges Hautgefühl, eine geringere Klebrigkeit sowie einen verbesserten Geruch.

Gegenstand der vorliegenden Erfindung sind daher Estergemische von mindestens einer gegebenenfalls verzweigten, gegebenenfalls ungesättigten, gegebenenfalls substituierten Carbonsäure mit einer Kettenlänge von 6 bis 30, insbesondere 8 bis 22, bevorzugt 10 bis 16 Kohlenstoffatomen mit 2-Methyl-1-butanol und 3-Methyl-1-butanol, wobei das Gewichtsverhältnis von 2-Methyl-1-butanol und 3-Methyl-1-butanol von 0,1 : 1 bis 1 : 1, bevorzugt von 0,1 : 1 bis 0,67: 1 beträgt.
wobei zur Herstellung der erfindungsgemäßen Ester als Säurekomponente Capronsäure, Caprylsäure, Caprinsäure, Laurinsäure, Myristinsäure, Palmitinsäure, Palmitoleinsäure, Isostearinsäure, 12-Hydroxystearinsäure, Dihydroxystearinsäure, Ölsäure, Linolsäure, Petrolesinsäure, Elaidinsäure, Arachinsäure, Behensäure, Erucasäure, Gadoleinsäure, Linolensäure, Eicosapentaensäure, Docosahexaensäure, Arachidonsäure, allein oder in Mischung eingesetzt werden, sowie Mischungen von Kokosfettsäuren, insbesondere vollhydrierten Kokosfettsäuren, besonders bevorzugt vollhydrierten Kokosfettsäuren mit einem Gehalt an Fettsäuren der Kettenlängen C12 bis C18 von zusammen >98 Gew.-% bezogen auf die Summe aller Carbonsäuren.

Besonders bevorzugt werden dabei einbasische, ggf. ungesättigte Fettsäuren mit 12-18 Kohlenstoffatomen, wie Laurinsäure, Myristinsäure, Palmitinsäure, Palmitoleinsäure, Isostearinsäure, Ölsäure, Linolsäure, Linolensäure eingesetzt, welche allein oder in Mischung eingesetzt werden können.

insbesondere bevorzugt wird dabei Laurinsäure, Myristinsäure, Palmitinsäure, welche allein oder in Mischung eingesetzt werden können.

Als Alkoholmischung wird eine Mischung aus 2-Methyl-1-butanol und 3-Methyl-1-butanol mit einem Gewichtsverhältnis von 2-Methyl-1-butanol zu 3-Methyl-1-butanol von 0,1 : 1 bis 1 : 1, bevorzugt von 0,1 : 1 bis 0,67 : 1, eingesetzt.

Das Mischungsverhältnis der eingesetzten Alkohole kann sowohl für diese Mischung als auch für die erhaltenen Veresterungsprodukte 1H-NMR spektrometrisch unter Verwendung von Standardmethoden und Standardlösungsmitteln (z. B. CDCl₃ oder DMSO-d6) bestimmt werden

Die Herstellung der Ester kann nach den dem Fachmann bekannten Methoden erfolgen, wie beispielsweise beschrieben im ORGANIKUM (Wiley-VCH, 22. Auflage 2004, Seite 474-480). Typische Verfahren zur Herstellung kosmetischer Ester nutzen Säuren, z. B. p-TSA, oder Metallsalze (z. B. Zinn- oder Titansalze) oder Enzyme (zum Beispiel Lipasen), als Katalysator, siehe z. B. EP1816154A1.

Die erfindungsgemäßen Estergemische lassen sich entweder wie in den Beispielen beschrieben durch Veresterung des Gemisches von 2-Methyl-1-butanol und 3-Methyl-1-butanol direkt erhalten; es ist aber erfindungsgemäß ebenfalls möglich, die Ester von 2-Methyl-1-butanol und 3-Methyl-1-butanol getrennt darzustellen und anschließend entsprechend abzumischen, wobei die erstgenannte Eintopfreaktion erfindungsgemäß bevorzugt ist.

Ein weiterer Gegenstand der vorliegenden Erfindung sind kosmetische, dermatologische oder pharmazeutische Formulierungen enthaltend ein erfindungsgemäßes Estergemisch, wobei
die erfindungsgemäßen Formulierungen als Wasser-in-Öl-, Öl-in-Wasser- oder Wasser-in-Silicon-Emulsionen hergestellt werden.

Ein weiterer Gegenstand der Erfindung ist die Verwendung der erfindungsgemäßen Estergemische zur Herstellung gegebenenfalls dispergierter Feststoffe enthaltender kosmetischer, dermatologischer oder pharmazeutischer Formulierungen oder Pflege- und Reinigungsmittel für Haushalt oder Industrie, insbesondere für harte Oberflächen, Leder oder Textilien. Somit sind die kosmetischen, dermatologischen oder pharmazeutischen Formulierungen, Pflege- und Reinigungsmittel für Haushalt oder Industrie und die Pflege- und Reinigungsmittel für harte Oberflächen, Leder oder Textilien enthaltend ein erfindungsgemäßes Estergemisch ebenfalls Gegenstand der vorliegenden Erfindung.

Die erfindungsgemäße Formulierung kann zum Beispiel mindestens eine zusätzliche Komponente enthalten, ausgewählt aus der Gruppe der
Emollients,
Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der EP2273966A1 entnommen werden. Diese Patentanmeldung wird hiermit als Referenz eingeführt und gilt somit als Teil der Offenbarung.

Bezüglich weiterer fakultativer Komponenten sowie die eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung. Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Alle angegebenen Prozent (%) sind wenn nicht anders angegeben Massenprozent.

### Beispiele:

### Beispiel 1: Herstellung von 2-Methyl-1-butyllaurat (Vergleichsbeispiel).

In einem Mehrhalsrundkolben werden 160 g 2-Methyl-1-butanol und 300 g Laurinsäure vorgelegt und auf 60 °C erhitzt. Nach Zugabe von 1,5 g Novozym 435 wird Vakuum angelegt (ca. 120 mbar) bis die Reaktionsmischung siedet. Das entstehende Reaktionswasser wird mit Hilfe eines Wasserabscheiders abgetrennt und der abdestillierte Alkohol zurück geführt. Nach 10 Stunden wird das immobilisierte Enzym abfiltriert und der überschüssige Alkohol destillativ entfernt. Durch anschließende Wasserdampfdestillation (2 Stunden, 120 °C) wird der Restgehalt an 2-Methyl-1-butanol auf 20 ppm reduziert. Das Produkt wird getrocknet (1 Stunde, 120°C, 20 mbar) und fällt ohne weitere Aufarbeitung als farblose Flüssigkeit an.

### Beispiel 2: Herstellung von 3-Methyl-1-butyllaurat (Vergleichsbeispiel).

In einem Mehrhalsrundkolben werden 160 g 3-Methyl-1-butanol und 300 g Laurinsäure vorgelegt und auf 60 °C erhitzt. Nach Zugabe von 1,5 g Novozym 435 wird Vakuum angelegt (ca. 120 mbar) bis die Reaktionsmischung siedet. Das entstehende Reaktionswasser wird mit Hilfe eines Wasserabscheiders abgetrennt und der abdestillierte zurück geführt. Nach 10 Stunden wird das immobilisierte Enzym abfiltriert und der überschüssige Alkohol destillativ entfernt. Durch anschließende Wasserdampfdestillation (2 Stunden, 120 °C) wird der Restgehalt an 3-Methyl-1-butanol auf 20 ppm reduziert. Das Produkt wird getrocknet (1 Stunde, 120°C, 20 mbar) und fällt ohne weitere Aufarbeitung als farblose Flüssigkeit an.

### Beispiel 3: Herstellung eines erfindungsgemäßen Gemischs von 2-Methyl-1-butyllaurat und 3-Methyl-1-butyllaurat.

In einem Mehrhalsrundkolben werden 64 g 2-Methyl-1-butanol, 96 g 3-Methyl-1-butanol und 300 g Laurinsäure vorgelegt und auf 60 °C erhitzt. Nach Zugabe von 1,5 g Novozym 435 wird Vakuum angelegt (ca. 120 mbar) bis die Reaktionsmischung siedet. Das entstehende Reaktionswasser wird mit Hilfe eines Wasserabscheiders abgetrennt und der abdestillierte Alkohol zurück geführt. Nach 10 Stunden wird das immobilisierte Enzym abfiltriert und der überschüssige Alkohol destillativ entfernt. Durch anschließende Wasserdampfdestillation (2 Stunden, 120 °C) wird der Restgehalt an 2-Methyl-1-butanol und 3-Methyl-1-butanol auf zusammen 20 ppm reduziert. Das Produkt wird getrocknet (1 Stunde, 120°C, 20 mbar) und fällt ohne weitere Aufarbeitung als farblose Flüssigkeit an.

### Beispiel 4: Herstellung von 2-Methyl-1-butylcocoat (Vergleichsbeispiel).

In einem Mehrhalsrundkolben werden 150 g 2-Methyl-1-butanol und 300 g Kokosfettsäure (vollhydrierte, destillierte Kokosfettsäure mit einem Gehalt an Fettsäuren der Kettenlängen C12 bis C18 von zusammen >98%) vorgelegt und auf 60 °C erhitzt. Nach Zugabe von 1,5 g Novozym 435 wird Vakuum angelegt (ca. 120 mbar) bis die Reaktionsmischung siedet. Das entstehende Reaktionswasser wird mit Hilfe eines Wasserabscheiders abgetrennt und der abdestillierte Alkohol zurück geführt. Nach 10 Stunden wird das immobilisierte Enzym abfiltriert und der überschüssige Alkohol destillativ entfernt. Durch anschließende Wasserdampfdestillation (2 Stunden, 120 °C) wird der Restgehalt an 2-Methyl-1-butanol auf 20 ppm reduziert. Das Produkt wird getrocknet (1 Stunde, 120°C, 20 mbar) und fällt ohne weitere Aufarbeitung als farblose Flüssigkeit an.

### Beispiel 5: Herstellung von 3-Methyl-1-butylcocoat (Vergleichsbeispiel).

In einem Mehrhalsrundkolben werden 150 g 3-Methyl-1-butanol und 300 g Kokosfettsäure (vollhydrierte, destillierte Kokosfettsäure mit einem Gehalt an Fettsäuren der Kettenlängen C12 bis C18 von zusammen >98%) vorgelegt und auf 60 °C erhitzt. Nach Zugabe von 1,5 g Novozym 435 wird Vakuum angelegt (ca. 120 mbar) bis die Reaktionsmischung siedet. Das entstehende Reaktionswasser wird mit Hilfe eines Wasserabscheiders abgetrennt und der abdestillierte Alkohol zurück geführt. Nach 10 Stunden wird das immobilisierte Enzym abfiltriert und der überschüssige Alkohol destillativ entfernt. Durch anschließende Wasserdampfdestillation (2 Stunden, 120°C) wird der Restgehalt an 3-Methyl-1-butanol auf 20 ppm reduziert. Das Produkt wird getrocknet (1 Stunde, 120°C, 20 mbar) und fällt ohne weitere Aufarbeitung als farblose Flüssigkeit an.

### Beispiel 6: Herstellung eines erfindungsgemäßen Gemischs von 2-Methyl-1-butylcocoat und 3-Methyl-1-butylcocoat.

In einem Mehrhalsrundkolben werden 64 g 2-Methyl-1-butanol, 96 g 3-Methyl-1-butanol und 300 g Kokosfettsäure (vollhydrierte, destillierte Kokosfettsäure mit einem Gehalt an Fettsäuren der Kettenlängen C12 bis C18 von zusammen >98%) vorgelegt und auf 60 °C erhitzt. Nach Zugabe von 1,5 g Novozym 435 wird Vakuum angelegt (ca. 120 mbar) bis die Reaktionsmischung siedet. Das entstehende Reaktionswasser wird mit Hilfe eines Wasserabscheiders abgetrennt und der abdestillierte Alkohol zurück geführt. Nach 10 Stunden wird das immobilisierte Enzym abfiltriert und der überschüssige Alkohol destillativ entfernt. Durch anschließende Wasserdampfdestillation (2 Stunden, 120 °C) wird der Restgehalt an 2-Methyl-1-butanol und 3-Methyl-1-butanol auf zusammen 20 ppm reduziert. Das Produkt wird getrocknet (1 Stunde, 120°C, 20 mbar) und fällt ohne weitere Aufarbeitung als farblose Flüssigkeit an.

### Beispiel 7: Herstellung eines erfindungsgemäßen Gemischs von 2-Methyl-1-butylcocoat und 3-Methyl-1-butylcocoat.

In einem Mehrhalsrundkolben werden 125 g 3-Methyl-1-butanol, 25 g 2-Methyl-1-butanol und 300 g Kokosfettsäure (vollhydrierte, destillierte Kokosfettsäure mit einem Gehalt an Fettsäuren der Kettenlängen C12 bis C18 von zusammen >98%) vorgelegt und auf 60 °C erhitzt. Nach Zugabe von 1,5 g Novozym 435 wird Vakuum angelegt (ca. 120 mbar) bis die Reaktionsmischung siedet. Das entstehende Reaktionswasser wird mit Hilfe eines Wasserabscheiders abgetrennt und der abdestillierte Alkohol zurück geführt. Nach 10 Stunden wird das immobilisierte Enzym abfiltriert und der überschüssige Alkohol destillativ entfernt. Durch anschließende Wasserdampfdestillation (2 Stunden, 120 °C) wird der Restgehalt an 2-Methyl-1-butanol und 3-Methyl-1-butanol auf zusammen 20 ppm reduziert. Das Produkt wird getrocknet (1 Stunde, 120°C, 20 mbar) und fällt ohne weitere Aufarbeitung als farblose Flüssigkeit an.

### Beispiel 8: Anwendungsbeispiele:

Alle Konzentrationen in den Anwendungsbeispielen sind in Gewichtsprozent angegeben. Zur Herstellung der Emulsionen wurden dem Fachmann bekannte übliche Homogenisierverfahren eingesetzt für Öl-in-Wasser und Wasser-in-Öl Emulsionen eingesetzt.

### Differenzierung der Performance gegen den Stand der Technik

Diese Versuche sollen zeigen, dass die erfindungsgemäßen Mischester aus 2-Methyl-1-butanol und 3-Methyl-1-butanol und Laurinsäure Vorteile in Bezug auf sensorische Eigenschaften und den Produktgeruch zu den jeweiligen reinen Estern bewirken.

### Beispiel 8. 1: Austestung von Lauratestern in O/W Lotionen:

Bei den Testformulierungen V1, V2 und 1 handelt es sich um O/W Lotionen. Dabei wurden Ölphase A und Wasserphase B jeweils auf 75°C erhitzt, zusammengegeben und anschließend mit einem geeigneten Homogenisator (z.B. Ultrathurrax) für ca. 1-2 Minuten homogenisiert.

Stabilisierende Polymere (Xanthan Gum, Carbomer) wurden als ölige Dispersion bei Temperaturen von 50 - 60°C in die Emulsion eingerührt. Anschließend wird eine Minute homogenisiert.

Zugabe weiterer Inhaltsstoffe (z.B. Konservierungsmittel, Wirkstoffe) erfolgt bevorzugt bei 40°C.

In den Formulierungsbeispielen sind die einzelnen Rohstoffe mit ihrer INCI-Bezeichnung aufgeführt.

**Tabelle 1: O/W Lotion Testformulierungen**

| | | V1 | V2 | 1 |
|---|---|---|---|---|
| A | Polyglyceryl-3 Dicitrate/Stearate¹⁾ | 2.5 | 2.5 | 2.5 |
| | Ester aus Beispiel 1 | 15.0 | - | - |
| | Ester aus Beispiel 2 | - | 15.0 | - |
| | Ester aus Beispiel 3 | - | - | 15.0 |
| B | Glycerin | 3.0 | 3.0 | 3.0 |
| | Demineralized Water | ad 100 | ad 100 | ad 100 |
| C | Carbomer | 0.1 | 0.1 | 0.1 |
| | Xanthan Gum | 0.3 | 0.3 | 0.3 |
| | Ethylhexyl Palmitate | 0.8 | 0.8 | 0.8 |
| D | NaOH 10% aq | q.s. | q.s. | q.s. |
| E | Phenoxyethanol, Ethylhexylglycerin²⁾ | 0.8 | 0.8 | 0.8 |

| | | | | |
|---|---|---|---|---|
| ¹⁾ TEGO^{®} Care PSC 3 (Evonik Goldschmidt GmbH) ²⁾ Euxyl^{®} PE 9010 (Schülke) | | | | |

Die Testformulierungen 1, V1 und V2 wurden durch ein geschulte sensorische Probandengruppe (N=13) untersucht. Das Hautgefühl der in den Beispielen beschriebenen kosmetischen Formulierungen wurde durch ein sogenanntes Panel bestimmt. Dabei wurden eine definierte Menge der Testlotionen auf ein klar definiertes Areal des Unterarms aufgetragen. Die Probanden verglichen die sensorischen Eigenschaften der kosmetischen Formulierungen und der jeweiligen Vergleichsformulierung ohne die Zusammensetzung zu kennen. Die Bewertung fand auf einer Skala 0 (wenig) bis 10 (viel) statt.

Während hinsichtlich von Anwendungseigenschaften wie "Verteilbarkeit", und "Gleitfähigkeit" keine Unterschiede zu den die Vergleichsemollients enthaltenden Formulierungen gefunden werden konnten, traten wahrnehmbare Unterschiede hinsichtlich der Eigenschaften "Absorption", "Öligkeit" und "Klebrigkeit" zu Tage. Außerdem bewertete die Gruppe den Geruch der Formulierungen auf einer Skala von 1 (gut) bis 3 (schlecht).

In Tabelle 2 sind die Mittelwerte der beschriebenen Austestung zusammengefasst.

**Tabelle2: Sensorischer und olfaktorischer Vergleich in O/W Testemulsionen**

| Formulierung | Verteilbarkeit | Absorption | Öligkeit | Klebrigkeit | Gleitfähigkeit | Geruch |
|---|---|---|---|---|---|---|
| V1 | 8 | 4 | 4 | 4 | 5 | 1,4 |
| V2 | 7 | 5 | 4 | 3 | 5 | 1,5 |
| 1 | 8 | 7 | 2 | 1 | 4 | 1,2 |

### Beispiel 8.2: Austestung von Cocoatestern in W/O Cremes:

Bei den Testformulierungen V3, V4 und 2 handelt es sich um W/O Cremes. Zur Herstellung wurde die Ölphase A auf 80°C erhitzt. Die Wasserphase wurde dann zur Ölphase langsam unter Rühren zugegeben und zusammengegeben und anschließend wurde mit einem geeigneten Homogenisator (z.B. Ultrathurrax) für ca. 1-2 Minuten homogenisiert.

Nach Zugabe des Konservierungsmittels (unterhalb von 40°C) wird unterhalb von 30°C noch einmal kurz homogenisiert.

**Tabelle 3: W/O Creme Testformulierungen**

| | | V3 | V4 | 2 |
|---|---|---|---|---|
| A | Diisostearoyl Polyglyceryl-3 Dimer Dilinoleate³⁾ | 3.0 | 3.0 | 3.0 |
| | Ester aus Beispiel 4 | 19.0 | - | - |
| | Ester aus Beispiel 5 | - | 19.0 | - |
| | Ester aus Beispiel 6 | - | - | 19.0 |
| | Hydrogenated Castor Oil | 0.5 | 0.5 | 0.5 |
| | Beeswax | 0.5 | 0.5 | 0.5 |
| B | Glycerin | 3.0 | 3.0 | 3.0 |
| | Demineralized Water | ad 100 | ad 100 | ad 100 |
| | Magnesium Sulfate Heptahydrate | 1.0 | 1.0 | 1.0 |
| D | NaOH 10% aq | q.s. | q.s. | q.s. |
| E | Sodium Benzoate, Potassium Benzoate, Aqua ⁴⁾ | 0.8 | 0.8 | 0.8 |

| | | | | |
|---|---|---|---|---|
| ³⁾ ISOLAN^{®} PDI (Evonik Goldschmidt GmbH) ⁴⁾ Euxyl^{®} K 712 (Schülke) | | | | |

In Tabelle 4 sind die Bewertungen des sensorischen und olfaktorischen Panels für die W/O Cremes zusammengefasst.

**Tabelle 4: Sensorischer und olfaktorischer Vergleich in W/O Testformulierungen**

| Formulierung | Verteilbarkeit | Absorption | Öligkeit | Klebrigkeit | Gleitfähigkeit | Geruch |
|---|---|---|---|---|---|---|
| V3 | 4 | 3 | 6 | 5 | 2 | 1,7 |
| V4 | 4 | 3 | 7 | 5 | 2 | 1,8 |
| 2 | 4 | 5 | 4 | 3 | 3 | 1,4 |

Der Vergleich in beiden Testemulsionen zeigt stimmig, dass die erfindungsgemäßen Mischester sensorische und olfaktorische Vorteile gegenüber den reinen Estern des Stands der Technik aufweisen.

### Beispiele zum Einsatz der erfindungsgemäßen Ester in kosmetischen Formulierungen

### Beispiel 9: Natürliche O/W Body Lotion

| | | |
|---|---|---|
| A | Polyglyceryl-3 Dicitrate/Stearate¹⁾ | 2.50 % |
| | Ester aus Beispiel 7 | 7.00 % |
| | Almond Oil | 5.00 % |
| | Caprylic/Capric Triglyceride²⁾ | 5.50 % |
| B | Water | 75.50 % |
| | Glycerin | 3.00 % |
| C | Xanthan Gum | 0.50 % |
| D | Sodium Hydroxide (10 %) | q. a. |
| E | Benzyl Alcohol; Glycerin; Benzoic Acid; Sorbic Acid³⁾ | 0.80 % |

| | | |
|---|---|---|
| 1) TEGO^{®} Care PSC 3 (Evonik Goldschmidt GmbH) 2) TEGOSOFT^{®} CT (Evonik Goldschmidt GmbH) 3) Rokonsal^{®} BSB-N (ISP) | | |

### Beispiel 10: Natürliche W/O Lotion

| | | |
|---|---|---|
| A | Polyglyceryl-4 Diisostearate/Polyhydroxystearate/Sebacate¹⁾ | 2.50 % |
| | Caprylic/Capric Triglyceride²⁾ | 7.50 % |
| | Ester aus Beispiel 7 | 6.00 % |
| | Jojoba Oil | 5.00 % |
| | Hyd. Castor Oil | 0.40 % |
| | Beeswax | 0.60 % |
| B | Water | 73.00 % |
| | Glycerin | 3.00 % |
| | Panthenol | 0.50 % |
| | Mg SO₄ × 7H₂O | 1.00 % |
| | Sodium Benzoate, Potassium Benzoate, Aqua³⁾ | 0.50 % |

| | | |
|---|---|---|
| ISOLAN^{®} GPS (Evonik Goldschmidt GmbH) TEGOSOFT^{®} CT(Evonik Goldschmidt GmbH) Euxyl^{®} K712 (Schülke & Mayr) | | |

### Beispiel 11: Natürliche O/W Crème

| | | |
|---|---|---|
| A | Polyglyceryl-3 Distearate/Stearate¹⁾ | 3.00 % |
| | Glyceryl Stearate²⁾ | 2.00 % |
| | Stearyl Alcohol³⁾ | 1.00 % |
| | Decyl Cocoate⁴⁾ | 2.90 % |
| | Ester aus Beispiel 7 | 5.00 % |
| | Oleyl Erucate⁵⁾ | 2.00 % |
| | Almond Oil | 10.00 % |
| | Ceramide III | 0.10 % |
| | Water | 70.20 % |
| B | Glycerin | 3.00 % |
| | Benzyl Alcohol; Glycerol, Benzoic Acid; Sorbic Acid⁶⁾ | 0.80 % |
| D | Sodium Hydroxide (10 %) | q. a. |

| | | |
|---|---|---|
| 1) TEGO^{®} Care PSC 3 (Evonik Goldschmidt GmbH) 2) TEGIN^{®} M (Evonik Goldschmidt GmbH) 3) TEGO^{®} Alkanol 18 (Evonik Goldschmidt GmbH) 4) TEGOSOFT^{®} DC (Evonik Goldschmidt GmbH)( 5) TEGOSOFT^{®} OER (Evonik Goldschmidt GmbH)( 6) Rokonsal^{®} BSB-N (ISP) | | |

### Beispiel 12: O/W Serum

| | | |
|---|---|---|
| A | Glyceryl Stearate; PEG-100 Stearate¹⁾ | 3.00 % |
| | Stearyl Alcohol²⁾ | 0.50 % |
| | Caprylic/Capric Triglyceride³⁾ | 5.00 % |
| | Cetyl Ricinoleate⁴⁾ | 1.00 % |
| | Ester aus Beispiel 7 | 5.50 % |
| B | Water | 76.125% |
| | Glycerin | 3.00 % |
| | Tetrapeptide-21; Glycerin; Butylene Glycol; Aqua⁵⁾ | 2.50 % |
| | Glycerin, Tetrapeptide-30⁶⁾ | 2.50 % |
| | Carbomer⁷⁾ | 0.0075% |
| C | Sodium Hydroxide (10 %) | q. a. |
| Z | Dipropylene Glycol; Methyl Paraben; Ethyl Paraben; Aqua; Methylisothiazolinone⁸⁾ | 0.80 % |

| | | |
|---|---|---|
| TEGO^{®} Care 165 (Evonik Goldschmidt GmbH) TEGO^{®} Alkanol 18 (Evonik Goldschmidt GmbH) TEGOSOFT^{®} CT (Evonik Goldschmidt GmbH) TEGOSOFT^{®} CR (Evonik Goldschmidt GmbH) TEGO^{®} PEP 4-17 (Evonik Goldschmidt GmbH) TEGO^{®} Pep 4 Even (Evonik Goldschmidt GmbH) TEGO^{®} Carbomer 141 G (Evonik Goldschmidt GmbH) Microcare^{®} MEM (Thor GmbH) | | |

### Beispiel 13: O/W Sonnenschutzlotion SPF 30

| | | |
|---|---|---|
| A | Glyceryl Stearate Citrate¹⁾ | 2.50 % |
| | Cetearyl Alcohol²⁾ | 1.00 % |
| | Caprylic/Captric Triglyceride³⁾ | 3.00 % |
| | Isopropyl Myristate⁴⁾ | 3.00 % |
| | Ester aus Beispiel 7 | 5.00 % |
| | Bis-Ethylhexyloxyphenol Methoxyphenyl Triazine⁵⁾ | 3.00 % |
| | Diethylamino Hydroxybenzoyl Hexyl Benzoate⁶⁾ | 2.50 % |
| | Octocrylene | 4.00 % |
| | Ethylhexyl Salicylate | 4.00 % |
| | Ethylhexyl Triazone | 1.50 % |
| B | Titanium Dioxide; Trimethoxycaprylylsilane⁷⁾ | 3.00 % |
| | Xanthan Gum | 0.20 % |
| | Carbomer⁸⁾ | 0.20 % |
| C | Glycerin | 3.00 % |
| | Water | 62.70 % |
| D | Sodium Hydroxide (10 %) | q. a. |
| Z | Dipropylene Glycol; Methyl Paraben; Ethyl Paraben; Aqua; Methylisothiazolinone⁹⁾ | 0.80 % |
| | Perfume | q. a. |

| | | |
|---|---|---|
| 1) AXOL^{®} C 62 (Evonik Goldschmidt GmbH) 2) TEGO^{®} Alkanol 1618 (Evonik Goldschmidt GmbH) 3) TEGOSOFT^{®} CT (Evonik Goldschmidt GmbH) 4) TEGOSOFT^{®} M (Evonik Goldschmidt GmbH) 5) Tinosorb^{®} S (BASF SE) 6) Uvinul^{®} A Plus (BASF SE) 7) TEGO^{®} Sun T 805 (Evonik Goldschmidt GmbH) 8) TEGO^{®} Carbomer 141 G(Evonik Goldschmidt GmbH) 9) Microcare^{®} MEM (Thor GmbH) | | |

### Beispiel 14: W/O Emulsion für ein Antiperspirant-Aerosol

| | | |
|---|---|---|
| A | Cetyl PEG/PPG-10/1 Dimethicone¹⁾ | 2.00 % |
| | Polyglyceryl-4 Diisostearate/Polyhydroxystearate/Sebacate²⁾ | 2.00 % |
| | Ester aus Beispiel 7 | 25.00 % |
| | C₂₋₁₅ Alkyl Benzoate³⁾ | 8.00 % |
| | Perfume | 3.00 % |
| B | Water | 34.20 % |
| | Aluminium Chlorohydrate | 20.00 % |
| | Propylenglycol | 5.00 % |
| C | Phenoxyethanol; Ethylhexylglycerol⁴⁾ | 0.80 % |

| | | |
|---|---|---|
| 1)ABIL^{®} EM 90 (Evonik Goldschmidt GmbH) 2) ISOLAN^{®} GPS (Evonik Goldschmidt GmbH) 3) TEGOSOFT^{®} TN (Evonik Goldschmidt GmbH) 4) Euxyl^{®} PE 9010 (Schülke & Mayr) | | |

### Beispiel 15: Wasserfreie Wasserfreies Konzentrat für Antiperspirant-Aerosol

| | | |
|---|---|---|
| A | Ester aus Beispiel 7 | 47.00 % |
| | Disteardimonium Hectorite | 3.00 % |
| | Diethylhexyl Carbonate¹⁾ | 10.00 % |
| | Silica Dimethyl Silylate²⁾ | 2.00 % |
| | Perfume | 5.00 % |
| B | C₂₋₁₅ Alkyl Benzoate³⁾ | 8.00 % |
| | Aluminium Chlorohydrate | 25.00 % |

| | | |
|---|---|---|
| TEGOSOFT^{®} DEC (Evonik Goldschmidt GmbH) AEROSIL^{®} R 972 (Evonik Degussa GmbH) TEGOSOFT^{®} TN (Evonik Goldschmidt GmbH) | | |

Das wasserfreie Konzentrat aus Beispiel 8 kann mit üblichen Treibgasenmischungen wie z.B. Propan/Butan/Isopropan/ Isobutan im Verhältnis von 25 bis 50 Teilen Emulsion zu 75 bis 50 Teilen Treibgas als Aerosol kombiniert werden.

### Beispiel 16: W/O Make-Up Formulierung

| | | |
|---|---|---|
| A | Polyglyceryl-4 Isostearate; Cetyl PEG/PPG-10/1 Dimethicone; Hexyl Laurate¹⁾ | 6,0 % |
| | Cetyl Dimethicone | 1,0 % |
| | Ester aus Beispiel 7 | 4,5 % |
| | Ethylhexyl Palmitate | 1,5 % |
| | Dimethicone | 5,0 % |
| | Cyclopentasiloxane | 9,0 % |
| | Phenyl Trimethicone | 1,0 % |
| | Lauryl Dimethicone/Polyglyceryl-3 Crosspolymer; Triethylhexanoin²⁾ | 2,0 % |
| | Nylon-12 | 1,0 % |
| | Iron Oxides | 2,0 % |
| | Titanium Oxide | 6,0 % |
| | Zinc Oxide | 0,5 % |
| B | Glycerin | 3,0 % |
| | Sodium Chloride | 0,8 % |
| | Creatine | 0,2 % |
| | Water | ad 100 % |
| Z | Preservative, Parfum | q.s. |

| | | |
|---|---|---|
| 1) ABIL^{®} WE 09 (Evonik Goldschmidt GmbH) 2) KSG-830 (Shin-Etsu) | | |

## Patentansprüche

1. Estergemisch von mindestens einer gegebenenfalls verzweigten, gegebenenfalls ungesättigten, gegebenenfalls substituierten Carbonsäure mit einer Kettenlänge von 6 bis 30, insbesondere 8 bis 22, bevorzugt 10 bis 16 Kohlenstoffatomen mit 2-Methyl-1-butanol und 3-Methyl-1-butanol, wobei das Gewichtsverhältnis von 2-Methyl-1-butanol und 3-Methyl-1-butanol von 0,1 : 1 bis 1 : 1, bevorzugt von 0,1 : 1 bis 0,67 : 1 beträgt, **dadurch gekennzeichnet, dass** die Carbonsäure ausgewählt ist aus der Gruppe:
Capronsäure, Caprylsäure, Caprinsäure, Laurinsäure, Myristinsäure, Palmitinsäure, Palmitoleinsäure, Isostearinsäure, 12-Hydroxystearinsäure, Dihydroxystearinsäure, Ölsäure, Linolsäure, Petrolesinsäure, Elaidinsäure, Arachinsäure, Behensäure, Erucasäure, Gadoleinsäure, Linolensäure, Eicosapentaensäure, Docosahexaensäure, Arachidonsäure, Mischungen von Kokosfettsäuren, insbesondere vollhydrierten Kokosfettsäuren, besonders bevorzugt vollhydrierten Kokosfettsäuren mit einem Gehalt an Fettsäuren der Kettenlängen C12 bis C18 von zusammen >98 Gew.-% bezogen auf die Summe aller Carbonsäuren.

2. Verwendung eines Estergemisches gemäß Anspruch 1 zur Herstellung kosmetischer, dermatologischer oder pharmazeutischer Formulierungen oder Pflege- und Reinigungsmittel für Haushalt oder Industrie.

3. Kosmetische, dermatologische oder pharmazeutische Formulierung oder Pflege- und Reinigungsmittel für Haushalt oder Industrie enthaltend ein Estergemisch gemäß Anspruch 1, **dadurch gekennzeichnet, dass** sie Wasser-in-Öl-, Öl-in-Wasser- oder Wasser-in-Silicon-Emulsionen sind.

## Claims

1. Ester mixture of at least one optionally branched, optionally unsaturated, optionally substituted carboxylic acid having a chain length of 6 to 30, in particular 8 to 22, preferably 10 to 16 carbon atoms, with 2-methyl-1-butanol and 3-methyl-1-butanol, wherein the ratio by weight of 2-methyl-1-butanol and 3-methyl-1-butanol is from 0.1:1 to 1:1, preferably from 0.1:1 to 0.67:1, **characterized in that** the carboxylic acid is selected from the group:
caproic acid, caprylic acid, capric acid, lauric acid, myristic acid, palmitic acid, palmitoleic acid, isostearic acid, 12-hydroxystearic acid, dihydroxystearic acid, oleic acid, linoleic acid, petroselinic acid, elaidic acid, arachidic acid, behenic acid, erucic acid, gadoleic acid, linolenic acid, eicosapentaenoic acid, docosahexaenoic acid, arachidonic acid, mixtures of coconut fatty acids, in particular fully hydrogenated coconut fatty acids, particularly preferably fully hydrogenated coconut fatty acids having a content of fatty acids of chain lengths C12 to C18 of together > 98% by weight, based on the sum of all carboxylic acids.

2. Use of an ester mixture according to Claim 1 for producing cosmetic, dermatological or pharmaceutical formulations or care and cleaning compositions for the home or industry.

3. Cosmetic, dermatological or pharmaceutical formulation or care and cleaning composition for the home or industry, comprising an ester mixture according to Claim 1, **characterized in that** said formulations/compositions are water-in-oil, oil-in-water or water-in-silicone emulsions.

## Revendications

1. Mélange d'esters d'au moins un acide carboxylique éventuellement ramifié, éventuellement insaturé, éventuellement substitué comportant une longueur de chaîne de 6 à 30, en particulier de 8 à 22, préférablement de 10 à 16 atomes de carbone avec le 2-méthyl-1-butanol et le 3-méthyl-1-butanol, le rapport en poids de 2-méthyl-1-butanol et de 3-méthyl-1-butanol étant de 0,1 : 1 à 1 : 1, préférablement de 0,1 : 1 à 0,67 : 1, **caractérisé en ce que** l'acide carboxylique est choisi dans le groupe :
acide caproïque, acide caprylique, acide caprique, acide laurique, acide myristique, acide palmitique, acide palmitoléique, acide isostéarique, acide 12-hydroxystéarique, acide dihydroxystéarique, acide oléique, acide linoléique, acide pétrosélinique, acide élaïdique, acide arachidique, acide béhénique, acide érucique, acide gadoléique, acide linolénique, acide eicosapentaénoïque, acide docosahexaénoïque, acide arachidonique, mélanges d'acides gras de coco, en particulier d'acides gras de coco entièrement hydrogénés, particulièrement préférablement d'acides gras de coco entièrement hydrogénés ayant une teneur en acides gras de longueurs de chaînes de C12 à C18 de > 98 % en poids au total par rapport à la somme de tous les acides carboxyliques.

2. Utilisation d'un mélange d'esters selon la revendication 1 pour la préparation de formulations cosmétiques, dermatologiques ou pharmaceutiques ou d'agents d'entretien et de nettoyage pour le ménage ou l'industrie.

3. Formulation cosmétique, dermatologique ou pharmaceutique ou agent d'entretien et de nettoyage pour le ménage ou l'industrie contenant un mélange d'esters selon la revendication 1, caractérisé(e) en ce que ce sont des émulsions eau-dans-huile, huile-dans-eau ou eau-dans-silicone.
